# FASCICULE DE BREVET EUROPEEN

(11) **EP 1 165 498 B1**
(45) Date de publication et mention de la délivrance du brevet: **20.08.2003**
(21) Numéro de dépôt: 00915244.8
(22) Date de dépôt: 29.03.2000
(51) Int. Cl.: C07C 253/30, C07C 255/24

(54) **PROCEDE D'HEMIHYDROGENATION DE DINITRILES EN AMINONITRILES**
VERFAHREN ZUR SELEKTIVEN HYDRIERUNG VON DINITRILEN ZU AMINONITRILEN
METHOD FOR HEMIHYDROGENATING DINITRILES TO FORM AMINONITRILES

(30) Priorité: 30.03.1999 FR 9904206
(43) Date de publication de la demande: 02.01.2002
(73) Titulaire: Rhodia Polyamide Intermediates, 69192 Saint-Fons (FR)
(72) Inventeur: DALLEMER, Frédéric, F-69003 Lyon (FR); SEIGNEURIN, Aline, F-78150 Le Chesnay (FR)
(74) Mandataire: Esson, Jean-Pierre
(86) Numéro de dépôt international: FR0000787
(87) Numéro de publication internationale: WO00059870

(56) Documents cités:
- EP-A- 0 077 911
- US-A- 4 536 347
- US-A- 5 512 697
- US-A- 5 756 808

## Description

La présente invention concerne l'hémihydrogénation de dinitriles en aminonitriles correspondants.

Généralement l'hydrogénation des dinitriles est réalisée pour préparer les diamines correspondantes ; ainsi particulièrement l'hydrogénation de l'adiponitrile conduit à l'hexaméthylène diamine, qui est elle-même l'un des monomères de base pour la préparation des polyamides.

Cependant il peut parfois s'avérer nécessaire de préparer non la diamine, mais l'aminonitrile intermédiaire. C'est par exemple, mais non limitativement le cas pour l'hémihydrogénation de l'adiponitrile en aminocapronitrile, susceptible d'être ensuite transformé soit en caprolactame, monomère de base pour la fabrication du polyamide-6 soit directement en polyamide-6.

Ainsi le brevet US 4 389 348 décrit un procédé d'hydrogénation de dinitrile en oméga-aminonitrile, par l'hydrogène, en milieu solvant aprotique et ammoniac et en présence de rhodium déposé sur un support basique.

Le brevet US 5 151 543 décrit un procédé d'hydrogénation partielle de dinitriles en aminonitriles dans un solvant en excès molaire d'au moins 2/1 par rapport au dinitrile, comprenant de l'ammoniac liquide ou un alcanol contenant une base minérale soluble dans ledit alcanol, en présence d'un catalyseur de type nickel ou cobalt de Raney.

Le brevet US 5 756 808 décrit également un procédé catalytique d'hémihydrogénation de dinitriles en aminonitrile. Les catalyseurs décrits sont à base de nickel, cobalt, fer, ruthénium ou rhodium éventuellement associés à d'autres éléments appelés éléments promoteurs tels que le palladium, le platine, l'iridium, l'osmium, le cuivre, l'argent, l'or, le chrome, le molybdène, le tungstène, le manganèse, le rhénium, le zinc, le cadmium, le plomb, l'aluminium, l'étain, le phosphore, l'arsenic, l'antimoine, le bismuth, et les éléments appartenant au groupe des terres rares. Ce document précise également que le ruthénium associé ou non avec le rhodium peut être utilisé sans les éléments promoteurs. Les catalyseurs sont déposés sur des supports tels que l'alumine, la silice. Les taux de sélectivité en aminocapronitrile (ACN) sont compris entre 70% et 80%.

Toutefois, pour obtenir un procédé industriel de préparation d'aminonitrile par hémihydrogénation d'un dinitrile, il est nécessaire d'obtenir des sélectivités encore plus élevées en aminonitrile pour des taux de transformation du dinitrile voisin de 100%, et donc de trouver de nouveaux systèmes catalytiques et des conditions de mise en oeuvre de l'hémihydrogénation permettant d'obtenir ou de se rapprocher de cet objectif.

Un des objets de la présente invention qui a trait à l'hydrogénation préférentielle d'une seule fonction nitrile d'un dinitrile (appelée dans le présent texte hémihydrogénation) de manière à préparer majoritairement l'aminonitrile correspondant et seulement de manière minoritaire la diamine, est notamment de proposer un nouveau système catalytique permettant d'améliorer le taux de sélectivité de cette réaction.

Plus précisément l'invention concerne un procédé d'hémihydrogénation de dinitriles aliphatiques en aminonitriles correspondants, à l'aide d'hydrogène et en présence d'un catalyseur du type catalyseur déposé ou adsorbé sur un support caractérisé en ce que le catalyseur est à base de ruthénium le support étant un charbon obtenu par pyrolyse d'une huile paraffinique. Ces charbons sont également appelés noir d'acétylène, terminologie que nous utiliserons dans la suite du texte.

Ainsi, ces noirs d'acétylène présentent une surface spécifique plus faible que celle des charbons actifs généralement utilisés comme support de catalyseur. Cette surface spécifique est généralement comprise entre 50 et 100 m²/g. Ces noirs d'acétylène se distinguent également par d'autres caractéristiques techniques telles que leur structure cristalline, leur état d'oxydation, une pureté chimique élevée et une grande porosité.

A titre d'exemple, on peut citer comme noirs d'acétylène convenables pour l'invention les noirs commercialisés par la société SN2A, sous les dénominations commerciales Y70, Y200, Y50, YS.

Les quantités de phases catalytiques présentes sur le support correspondent aux quantités usuellement déposées dans les systèmes catalytiques supportés.

L'utilisation du support particulier de l'invention permet d'obtenir une sélectivité élevée en aminonitriles pour des taux de transformation élevés des dinitriles. Ce niveau de sélectivité ne peut être atteint en utilisant d'autres supports classiques tels que l'alumine, la silice ou les charbons actifs généralement d'origine végétale.

Ainsi, l'utilisation de ce système catalytique permet d'atteindre un taux de sélectivité en aminonitriles d'au moins 80% pour un taux de transformation du dinitrile supérieur à 60%. Ce taux de sélectivité est supérieur à celui obtenu avec un catalyseur Nickel de Raney.

Selon une nouvelle caractéristique préférentielle de l'invention, le catalyseur peut être soumis à une réduction par un agent réducteur tel que l'hydrogène, par exemple, avant introduction dans le milieu réactionnel.

Selon un autre mode de réalisation de l'invention, le catalyseur comprend des éléments dopants dont au moins un est du fer.

La présence de ces éléments dopant et notamment du fer permet d'augmenter la durée de vie du catalyseur et donc l'économie du procédé d'hémihydrogénation.

La concentration en élément dopant par rapport au ruthénium est comprise entre 5% et 60% en poids, de préférence entre 10 et 30% en poids.

Les éléments dopants autre que le fer convenables pour l'invention sont choisis dans le groupe comprenant le tungstène, le manganèse, le rhénium, le zinc, le cadmium, le plomb, l'aluminium, l'étain, le phosphore, l'arsenic, l'antimoine, le bismuth, le silicium, le titane, le zirconium, les terres rares, le palladium, le platine, l'iridium, l'osmium, le cuivre, l'argent, le chrome, le molybdène.

Les dinitriles aliphatiques qui peuvent être mis en oeuvre dans le procédé de l'invention sont plus particulièrement les dinitriles de formule générale (I) :

NC―R―CN (I)

dans laquelle R représente un groupement alkylène ou alcénylène, linéaire ou ramifié, ayant de 1 à 12 atomes de carbone.

De préférence, on met en oeuvre dans le procédé de l'invention des dinitriles de formule (I) dans laquelle R représente un radical alkylène, linéaire ou ramifié ayant de 2 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

Le composé préféré de l'invention est l'adiponitrile qui permet d'obtenir un précurseur du polyamide 6 ou de son monomère le caprolactame.

Selon un mode de réalisation préféré de l'invention, la réaction d'hémihydrogénation est réalisée en présence d'ammoniac. Cette caractéristique permet d'améliorer les performances du système catalytique de l'invention, notamment sa durée de vie.

Selon une autre caractéristique préférentielle de l'invention, l'hemihydrogénation d'un dinitrile en aminonitrile peut être réalisée en présence d'une base minérale forte dérivant d'un élément alcalin, alcalino-terreux ou du cation ammonium.

Ainsi, la base minérale forte est généralement constituée par les hydroxydes, les carbonates et les alcanolates de métal alcalin, de métal alcalino-terreux ou du cation ammonium. Elle est choisie de préférence parmi les hydroxydes, les carbonates et les alcanolates de métal alcalin.

De façon privilégiée, la base minérale forte mise en oeuvre est choisie parmi les composés suivants : LiOH, NaOH, KOH, RbOH, CsOH , les composés comprenant un radical ammonium quaternaire, et les mélanges de ces composés. En pratique, on utilise généralement NaOH et KOH.

Ces bases fortes sont présentes dans le milieu réactionnel selon une concentration comprise entre 0,1 et 50 mole de OH⁻ par Kg de système catalytique, de préférence entre 5 et 35 mole de OH⁻ par Kg de système catalytique. En outre, cette concentration est beaucoup plus faible voire nulle quand l'hydrogénation est réalisée en présence d'ammoniac.

La composition du milieu réactionnel d'hydrogénation est variable et dépend notamment du mode de conduite de celui-ci.

En effet, si le procédé est mise en oeuvre en mode discontinu, le milieu réactionnel initial s'enrichit progressivement en aminonitrile et, en moindre proportion, en diamine, tandis que la concentration en dinitrile peut soit décroître si l'on charge la totalité ou la majeure partie dudit dinitrile dès le début de l'hémihydrogénation, soit demeurer relativement constante si le dinitrile est introduit progressivement pendant la réaction.

Par contre, si le procédé est conduit en mode continu, la composition moyenne du milieu réactionnel est sensiblement constante, en dehors des périodes de démarrage de l'installation.

Le milieu réactionnel peut comprendre de l'eau dans des quantités comprise dans de très larges limites. Ainsi, le milieu peut contenir jusqu'à 50% en poids d'eau et même au-delà. Préférentiellement, la teneur en eau du milieu réactionnel est comprise entre 2 % et 15 % en poids par rapport à l'ensemble des constituants liquides dudit milieu.

De préférence, la concentration en composés aminonitrile, diamine et dinitrile dans le milieu réactionnel est généralement comprise entre 85 % et 98 % en poids par rapport à l'ensemble des constituants liquides dudit milieu réactionnel à l'exclusion de l'ammoniac quand il est présent.

En fonctionnement continu du procédé de l'invention, la composition moyenne sera déterminée par le rapport des sélectivités respectives en aminonitrile et en diamine et par le débit d'introduction du dinitrile.

La quantité de catalyseur mise en oeuvre dans le milieu réactionnel, peut varier très largement en fonction notamment du mode de fonctionnement adopté ou des conditions réactionnelles choisies. Ainsi, si l'on introduit progressivement le dinitrile dans le milieu réactionnel, le rapport pondéral catalyseur/dinitrile à hydrogéner sera beaucoup plus élevé que si tout le dinitrile est mis en oeuvre dès le début de la réaction. A titre indicatif, on peut utiliser de 0,5 % à 50 % en poids de catalyseur par rapport au poids total du milieu réactionnel et le plus souvent de 1 % à 35 % .

Le procédé de l'invention est généralement mis en oeuvre à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 1bar (0,10 MPa) et 300 bar (30 MPa).

La durée de la réaction est variable en fonction des conditions réactionnelles et du catalyseur.

Dans un mode de fonctionnement discontinu, elle peut varier de quelques minutes à plusieurs heures.

Il est à noter que l'homme du métier peut moduler la chronologie des étapes du procédé selon l'invention, selon les conditions opératoires.

Les autres conditions qui régissent l'hydrogénation (en mode continu ou discontinu) conforme à l'invention, relèvent de dispositions techniques traditionnelles et connues en elles-mêmes.

Les exemples qui suivent donnés uniquement à titre indicatif, illustrent l'invention.

Dans ces exemples les abréviations suivantes sont utilisées :
- ADN = adiponitrile
- ACN = aminocapronitrile
- HMD = hexaméthylène diamine
- TT = taux de transformation
- S_{(ACN)} = sélectivité en ACN par rapport au substrat de départ transformé
(ici par rapport à l'ADN).

### Exemple 1 : Préparation d'un catalyseur 5%Ru-1%Fe sur support noir d'acétylène Y70

On charge 20 g de noir d'acétylène Y70 commercialisé par SN2A, dans 800 ml d'eau. La suspension est chauffée à 90°C, sous agitation. On additionne 1,8 g de Na₂CO₃, dans un total de 70 ml d'eau. Après une période de 1 heure, on additionne une solution de 2,16 g de RuCl₃ hydraté dans 120 ml d'eau. Après 1 heure, on coule une solution de 1 g de FeCl₃ hexahydraté dans un total de 70 ml d'eau, puis après une nouvelle heure, on laisse refroidir le milieu jusqu'à une température de 40°C. Après filtration, le catalyseur est lavé avec 4 fois 200 ml d'eau à 40°C.

Le catalyseur est séché en étuve durant 1 heure, à 120°C. On obtient 21,3 g de catalyseur.

Avant test, il est séché en étuve durant 10 heures, à 80°C, sous pression réduite.

### Exemple 2 : Préparation d'un catalyseur 5%Ru-0.5%Fe sur noir d'acétylène Y200

Le support noir d'acétylène commercialisé par la société SN2A sous la dénomination Y200, est préalablement calciné sous air durant 1 heure, à 500°C. On charge 20 g du noir d'acétylène Y200 calciné, dans 800 ml d'eau. La suspension est chauffée à 90°C, sous agitation. On additionne 1,8 g de Na₂CO₃, dans un total de 70 ml d'eau. Après une période de 1 heure, on additionne une solution de 2,16 g de RuCl₃ hydraté dans 90 ml d'eau. Après 1 heure, on coule une solution de 0,51 g de FeCl₃ hexahydraté dans un total de 70 ml d'eau.

Après 1 heure, on laisse refroidir le milieu jusqu'à une température de 40°C. Après filtration, le catalyseur est lavé avec 4 fois 200 ml d'eau à 40°C.

Le catalyseur est séché en étuve durant 1 heure, à 120°C. On obtient 21,2 g de catalyseur.

Il est séché avant test, en étuve durant 10 heures, à 80°C, sous pression réduite.

### Exemple 3 : Préparation d'un catalyseur 5%Ru-1%Fe sur support noir d'acétylène Y200

Le support noir d'acétylène Y200 est préalablement calciné sous air durant 1 heure, à 500°C.

On charge 20 g du noir d'acétylène Y200 calciné, dans 800 ml d'eau. La suspension est chauffée à 90°C, sous agitation. On additionne 2 g de Na₂CO₃, dans un total de 70 ml d'eau. Après une période de 1 heure, on additionne une solution de 2,16 g de RuCl₃ hydraté dans 90 ml d'eau. Après 1 heure, on coule une solution de 1 g de FeCl₃ hexahydraté dans un total de 70 ml d'eau.

On laisse refroidir le milieu jusqu'à une température de 4°C.

Après filtration, le catalyseur est lavé avec 4 fois 200 ml d'eau à 40°C.

Le catalyseur est séché en étuve durant 1 heure, à 120°C. On obtient 21,5 g de catalyseur.

Il est réduit avant test, sous hydrogène, à une température s'élevant de la température ambiante jusqu'à 100°C et maintenu 4 heures à cette température.

### Exemple 4 : Hémihydrogénation d'adiponitrile

Dans un réacteur, équipé d'une agitation, de moyen d'introduction de l'hydrogène et d'un système de régulation de température, on charge :
- catalyseur, selon exemple 1 2,4 g
- ADN 36 g
- HMD 36 g
- KOH 15 N dans l'eau 5 g
- eau 4,8 g

On chauffe le mélange réactionnel à 80°C après avoir purgé le réacteur à l'azote, puis à l'hydrogène ; la pression est réglée à 2,5 MPa à cette température par addition continue d'hydrogène. L'avancement de la réaction est suivi par la consommation d'hydrogène et l'analyse par chromatographie phase vapeur (CPG) d'un prélèvement du mélange réactionnel.

On obtient les résultats suivants :
- temps de réaction : 105 min
- TT de l'ADN : 67 %
- S(ACN) 75 %

### Exemple 5: Hémihydrogénation d'adiponitrile

Dans un réacteur de 150 ml, équipé d'une agitation, de moyen d'introduction de l'hydrogène et d'un système de régulation de température, on charge :
- catalyseur, selon exemple 2 1,5 g
- ADN 22 g
- HMD 22 g
- KOH 15 N dans l'eau 3,1 g
- eau 3 g

On chauffe le mélange réactionnel à 80°C après avoir purgé le réacteur à l'azote, puis à l'hydrogène ; la pression est réglée à 2,5 MPa à cette température par addition continue d'hydrogène. L'avancement de la réaction est suivi par la consommation d'hydrogène et l'analyse par chromatographie phase vapeur (CPG) d'un prélèvement du mélange réactionnel.

On obtient les résultats suivants :
- temps de réaction : 90 min
- TT de l'ADN : 85 %
- S(ACN) 73 %

### Exemple. 6 : Hémihydrogénation d'adiponitrile en présence de NH₃

Dans un réacteur de 300 ml, équipé d'une agitation, de moyen d'introduction de l'hydrogène et d'un système de régulation de température, on charge :
- catalyseur, selon exemple 3 4 g
- ADN 42,5 g
- eau 10,6 g
- NH3 64,4 g

On chauffe le mélange réactionnel à 80°C après avoir purgé le réacteur à l'azote, puis à l'hydrogène ; la pression est réglée à 11 MPa à cette température par addition continue d'hydrogène. L'avancement de la réaction est suivi par la consommation d'hydrogène et l'analyse par chromatographie phase vapeur (CPG) d'un prélèvement du mélange réactionnel.

On obtient les résultats suivants :
- temps de réaction : 300 min
- TT de l'ADN : 57 %
- S(ACN) 82 %

### Exemple A comparatif : Hémihydrogénation d'adiponitrile

Dans un réacteur de 300 ml, équipé d'une agitation, de moyen d'introduction de l'hydrogène et d'un système de régulation de température, on charge :
- Ni de Raney (dopé par du fer et du chrome) 3 g
- eau 13,5 g
- KOH 1 N dans l'eau 1,5 g
- ADN 67,5 g
- HMD 67,5 g

On chauffe le mélange réactionnel à 50°C après avoir purgé le réacteur à l'azote, puis à l'hydrogène ; la pression est réglée à 2,2 MPa à cette température par addition continue d'hydrogène. L'avancement de la réaction est suivi par la consommation d'hydrogène et l'analyse par chromatographie phase vapeur (CPG) d'un prélèvement du mélange réactionnel.

On obtient les résultats suivants :
- temps de réaction : 15 min
- TT de l'ADN : 66 %
- S(ACN) 80 %

### Exemple B comparatif : Hémihydrogénation d'adiponitrile

Dans un réacteur de 180 ml, équipé d'une agitation, de moyen d'introduction de l'hydrogène et d'un système de régulation de température, on charge :
- catalyseur 5%Ru sur support charbon actif commercialisé par Engelhard sous la dénomination ESCAT 40 2,4 g
- ADN 36 g
- HMD 36 g
- KOH 15 N dans l'eau 0,9 g
- eau 8,8 g

Le catalyseur a préalablement été réduit sous hydrogène, à une température s'élevant de la température ambiante jusqu'à 500°C et maintenu 4 heures à cette température.

On chauffe le mélange réactionnel à 80°C après avoir purgé le réacteur à l'azote, puis à l'hydrogène ; la pression est réglée à 2,5 MPa à cette température par addition continue d'hydrogène. L'avancement de la réaction est suivi par la consommation d'hydrogène et l'analyse par chromatographie phase vapeur (CPG) d'un prélèvement du mélange réactionnel.

On obtient les résultats suivants :
- temps de réaction : 120 min
- TT de l'ADN : 73 %
- S(ACN) 36 %

## Revendications

1. Procédé d'hémihydrogénation de dinitriles en aminonitriles correspondants, l'aide d'hydrogène et en présence d'un système catalytique du type catalyseur supporté **caractérisé en ce que** le catalyseur comprend du ruthénium supporté sur un noir de carbone, appelé noir d'acétylène, issu de la pyrolyse d'huiles paraffiniques.

2. Procédé selon la revendication 1, **caractérisé en ce que** le système catalytique comprend au moins un élément dopant dont au moins un est le fer.

3. Procédé selon la revendication 2, **caractérisé en ce que** la concentration en élément dopant par rapport au ruthénium est comprise entre 5% et 60% en poids de préférence entre 10 et 30% en poids.

4. Procédé selon l'une des revendications précédentes, **caractérisé en ce que** le catalyseur est soumis à une réduction préalablement à son utilisation.

5. Procédé selon l'une des revendications 2 à 4, **caractérisé en ce que** les éléments dopants autre que le fer sont choisis dans le groupe comprenant le tungstène, le manganèse, le rhénium, le zinc, le fer, le cadmium, le plomb, l'aluminium, l'étain, le phosphore, l'arsenic, l'antimoine, le bismuth, le silicium, le titane, le zirconium les terres rares, le palladium, le platine, l'iridium, l'osmium, le cuivre, l'argent, le chrome, le molybdène.

6. Procédé selon l'une des revendications précédentes, **caractérisé en ce que** le milieu initial d'hydrogénation contient de l'ammoniac

7. Procédé selon l'une des revendications précédentes, **caractérisé en ce que** le milieu initial d'hydrogénation contient une base minérale forte choisie parmi les hydroxydes, les carbonates et les alcanolates de métal alcalin, de métal alcalino-terreux ou du cation ammonium, et de préférence parmi les hydroxydes, les carbonates et les alcanolates de métal alcalin, les composés comprenant un radical ammonium quaternaire.

8. Procédé selon la revendication 7, **caractérisé en ce que** la base minérale forte mise en oeuvre est choisie parmi les composés suivants : LiOH, NaOH, KOH, RbOH, CsOH et leur mélanges.

9. Procédé selon l'une des revendications 7 ou 8, **caractérisé en ce que** la quantité, exprimée en mole de OH⁻, de base minérale présente dans le milieu réactionnel est comprise entre 0,1 mole par kilogramme de catalyseur et 50 mole/Kg, de préférence comprise entre 5 et 35 mole par kilogramme de catalyseur.

10. Procédé selon l'une des revendications précédentes, **caractérisé en ce que** les dinitriles aliphatiques qui peuvent être mis en oeuvre dans le procédé de l'invention sont les dinitriles de formule générale (I) :
NC―R―CN (I)
dans laquelle R représente un groupement alkylène ou alcénylène, linéaire ou ramifié, ayant de 1 à 12 atomes de carbone et de préférence R représente un radical alkylène, linéaire ou ramifié ayant de 2 à 6 atomes de carbone.

11. Procédé selon l'une des revendications précédentes, **caractérisé en ce que** la concentration de l'aminonitrile visé et/ou de la diamine correspondante et du dinitrile non transformé dans le milieu réactionnel est comprise entre 80 % et 99,5 % en poids par rapport à l'ensemble des liquides inclus dans ledit milieu réactionnel, à l'exclusion de l'ammoniac quand il est présent.

12. Procédé selon l'une des revendications 1 à 11, **caractérisé en ce qu'**il est mis en oeuvre à une température réactionnelle inférieure ou égale à 150°C, de préférence inférieure ou égale à 120°C et, plus préférentiellement encore, inférieure ou égale à 100°C.

13. Procédé selon l'une des revendications 1 à 12, **caractérisé en ce que** le l'on opère à une pression en hydrogène comprise entre 1 bar (0,10 MPa) et 300 bar (30 Mpa).

## Patentansprüche

1. Verfahren zur Hemihydrierung von Dinitrilen zu entsprechenden Aminonitrilen mit Hilfe von Wasserstoff und in Gegenwart eines katalytischen Systems vom Typ trägergebundener Katalysator, **dadurch gekennzeichnet, dass** der Katalysator Ruthenium umfasst, das auf einem Kohlenstoffruß, genannt Acetylenruß, gebunden ist, der aus der Pyrolyse von Paraffinölen stammt.

2. Verfahren gemäß Anspruch 1, **dadurch gekennzeichnet, dass** das katalytische System wenigstens ein Dotierungselement umfasst, von dem wenigstens eins Eisen ist.

3. Verfahren gemäß Anspruch 2, **dadurch gekennzeichnet, dass** die Konzentration an Dotierungselement, bezogen auf das Ruthenium, zwischen 5 Gew.-% und 60 Gew.-%, vorzugsweise zwischen 10 und 30 Gew.-% liegt.

4. Verfahren gemäß einem der vorhergehenden Ansprüche, **dadurch gekennzeichnet, dass** der Katalysator vor seiner Verwendung einer Reduktion unterzogen wird.

5. Verfahren gemäß einem der Ansprüche 2 bis 4, **dadurch gekennzeichnet, dass** die von Eisen verschiedenen Dotierungselemente aus der Gruppe ausgewählt sind, die Wolfram, Mangan, Rhenium, Zink, Eisen, Cadmium, Blei, Aluminium, Zinn, Phosphor, Arsen, Antimon, Bismut, Silicium, Titan, Zirkonium, die Seltenen Erden, Palladium, Platin, Iridium, Osmium, Kupfer, Silber, Chrom, Molybdän umfasst.

6. Verfahren gemäß einem der vorhergehenden Ansprüche, **dadurch gekennzeichnet, dass** das anfängliche Hydrierungsmedium Ammoniak enthält.

7. Verfahren gemäß einem der vorhergehenden Ansprüche, **dadurch gekennzeichnet, dass** das anfängliche Hydrierungsmedium eine starke mineralische Base enthält, die ausgewählt ist unter den Hydroxiden, den Carbonaten und den Alkanolaten eines Alkalimetalls, eines Erdalkalimetalls oder des Ammoniumkations und vorzugsweise unter den Hydroxiden, den Carbonaten und den Alkanolaten eines Alkalimetalls, den Verbindungen, die einen quartären Ammoniumrest umfassen.

8. Verfahren gemäß Anspruch 7, **dadurch gekennzeichnet, dass** die eingesetzte starke mineralische Base ausgewählt ist unter den folgenden Verbindungen: LiOH, NaOH, KOH, RbOH, CsOH und ihren Gemischen.

9. Verfahren gemäß einem der Ansprüche 7 oder 8, **dadurch gekennzeichnet, dass** die Menge, ausgedrückt in Mol OH⁻, an mineralischer Base, die in dem Reaktionsmedium vorhanden ist, zwischen 0,1 Mol pro Kilogramm Katalysator und 50 mol/kg. vorzugsweise zwischen 5 und 35 Mol pro Kilogramm Katalysator liegt.

10. Verfahren gemäß einem der vorhergehenden Ansprüche, **dadurch gekennzeichnet, dass** die aliphatischen Dinitrile, die bei dem Verfahren der Erfindung eingesetzt werden können, die Dinitrile der allgemeinen Formel (I):
NC-R-CN (I)
sind, worin R eine lineare oder verzweigte Alkylen- oder Alkenylengruppe mit 1 bis 12 Kohlenstoffatomen darstellt und R vorzugsweise einen linearen oder verzweigten Alkylenrest mit 2 bis 6 Kohlenstoffatomen darstellt.

11. Verfahren gemäß einem der vorhergehenden Ansprüche, **dadurch gekennzeichnet, dass** die Konzentration des angestrebten Aminonitrils und/oder des entsprechenden Diamins und des nicht umgewandelten Dinitrils in dem Reaktionsmedium zwischen 80 Gew.-% und 99,5 Gew.-%, bezogen auf die Gesamtheit der in besagtem Reaktionsmedium enthaltenen Flüssigkeiten, mit Ausnahme des Ammoniaks, wenn er vorhanden ist, liegt.

12. Verfahren gemäß einem der Ansprüche 1 bis 11, **dadurch gekennzeichnet, dass** es bei einer Reaktionstemperatur kleiner oder gleich 150 °C, vorzugsweise kleiner oder gleich 120 °C und noch stärker bevorzugt kleiner oder gleich 100 °C eingesetzt wird.

13. Verfahren gemäß einem der Ansprüche 1 bis 12, **dadurch gekennzeichnet, dass** man bei einem Wasserstoffdruck zwischen 1 bar (0,10 MPa) und 300 bar (30 MPa) arbeitet.

## Claims

1. Process for the hemihydrogenation of dinitriles to corresponding aminonitriles using hydrogen in the presence of a catalytic system of the supported catalyst type, **characterized in that** the catalyst comprises ruthenium supported on a carbon black, called acetylene black, resulting from the pyrolysis of paraffin oils.

2. Process according to Claim 1, **characterized in that** the catalytic system comprises at least one doping element, at least one of which is iron.

3. Process according to Claim 2, **characterized in that** the concentration of doping element with respect to the ruthenium is between 5% and 60% by weight, preferably between 10 and 30% by weight.

4. Process according to one of the preceding claims, **characterized in that** the catalyst is subjected to reduction prior to its use.

5. Process according to one of Claims 2 to 4, **characterized in that** the doping elements other than iron are chosen from the group consisting of tungsten, manganese, rhenium, zinc, iron, cadmium, lead, aluminium, tin, phosphorus, arsenic, antimony, bismuth, silicon, titanium, zirconium, the rare earth metals, palladium, platinum, iridium, osmium, copper, silver, chromium and molybdenum.

6. Process according to one of the preceding claims, **characterized in that** the starting hydrogenation mixture comprises ammonia.

7. Process according to one of the preceding claims, **characterized in that** the starting hydrogenation mixture comprises a strong inorganic base chosen from alkali metal or alkaline earth metal hydroxides, carbonates and alkoxides or hydroxides, carbonates and alkoxides of the ammonium cation and preferably from alkali metal hydroxides, carbonates and alkoxides or compounds comprising a quaternary ammonium radical.

8. Process according to Claim 7, **characterized in that** the strong inorganic base employed is chosen from the following compounds: LiOH, NaOH, KOH, RbOH, CsOH and their mixtures.

9. Process according to either of Claims 7 and 8, **characterized in that** the amount, expressed in moles of OH⁻, of inorganic base present in the reaction mixture is between 0.1 mol per kilogram of catalyst and 50 mol/kg, preferably between 5 and 35 mol per kilogram of catalyst.

10. Process according to one of the preceding claims, **characterized in that** the aliphatic dinitriles which can be employed in the process of the invention are the dinitriles of general formula (I) :
NC-R-CN (I)
in which R represents a linear or branched alkylene or alkenylene group having from 1 to 12 carbon atoms and R preferably represents a linear or branched alkylene radical having from 2 to 6 carbon atoms.

11. Process according to one of the preceding claims, **characterized in that** the concentration of the targeted aminonitrile and/or of the corresponding diamine and of the unconverted dinitrile in the reaction mixture is between 80% and 99.5% by weight with respect to all the liquids included in the said reaction mixture, with the exception of the ammonia, when it is present.

12. Process according to one of Claims 1 to 11, **characterized in that** it is carried out at a reaction temperature of less than or equal to 150°C, preferably of less than or equal to 120°C and more preferably still of less than or equal to 100°C.

13. Process according to one of Claims 1 to 12, **characterized in that** it is carried out at a hydrogen pressure of between 1 bar (0.10 MPa) and 300 bar (30 Mpa).
